Europäisches Patentamt

European Patent Office

Office européen des brevets

(11) Numéro de publication : **0 049 723**
**B1**

## (12) FASCICULE DE BREVET EUROPÉEN

(45) Date de publication du fascicule du brevet :
28.05.86

(21) Numéro de dépôt : 80401465.2

(22) Date de dépôt : 14.10.80

(51) Int. Cl.⁴ : **G 01 L 27/00**, **A 61 B 5/02**,
**F 15 B 21/12**

(54) **Procédé de vérification d'un système de mesure de pression et appareil pour réaliser ledit procédé.**

(43) Date de publication de la demande :
21.04.82 Bulletin 82/16

(45) Mention de la délivrance du brevet :
28.05.86 Bulletin 86/22

(84) Etats contractants désignés :
AT BE CH DE GB IT LI LU NL SE

(56) Documents cités :
DE-A- 2 057 217
FR-A- 1 556 606

(73) Titulaire : **Cieutat, Bertrand**
**2, rue Marie Benoist**
**F-75012 Paris 12 (FR)**

(72) Inventeur : **Cieutat, Bertrand**
**2, rue Marie Benoist**
**F-75012 Paris 12 (FR)**

(74) Mandataire : **Rataboul, Michel**
**Cabinet Michel Rataboul 69, rue de Richelieu**
**F-75002 Paris (FR)**

EP 0 049 723 B1

Jouve, 18, rue St-Denis, 75001 Paris, France

## Description

La présente invention concerne un appareil générateur d'une pression fluide variable, ces variations présentant elles-mêmes une fréquence variable.

Un tel appareil sera plus particulièrement destiné, à titre d'exemple, à la vérification d'appareils de mesure de pression sanguine.

Il est usuel, en technique médicale et particulièrement en chirurgie, d'avoir à mesurer la pression sanguine, et surtout les variations de cette pression, à l'intérieur d'un vaisseau ou d'un organe profond. Pour de telles investigations, on utilise couramment des capteurs consistant en tubes souples ou « cathéters », de différents diamètres, épaisseurs ou longueurs. Ces tubes sont remplis d'un liquide de transmission de pression, puis introduits par un vaisseau externe jusqu'à un vaisseau plus interne, ou à l'organe du patient dont on veut étudier le fonctionnement. L'extrémité libre du cathéter est raccordée à un appareil usuel de mesure de pression à membrane élastique, où les déplacements de la membrane traduisent les variations de pression sanguine à l'autre extrémité engagée dans le corps du patient. Ces informations sur les variations de pression sont de manière tout à fait usuelle visualisées sur un écran, ou enregistrées sous forme de courbes, ou même directement traitées en données électroniques.

Mais un tel système de transmission constitué par le cathéter rempli de liquide en relation avec une membrane élastique, présente une fréquence propre d'oscillation dont la valeur est tout à fait aléatoire. Elle peut dépendre en effet de très nombreux facteurs tels que la rigidité de la membrane, l'élasticité du tube, fonction elle-même de son épaisseur, de son diamètre, de sa longueur, ou d'irrégularités locales d'épaisseur ; elle peut encore dépendre de la viscosité du liquide de remplissage ou de la présence de bulles d'air. Le système peut alors entrer en résonance si la fréquence de la pulsation sanguine correspond à sa fréquence propre d'oscillation, et la courbe d'enregistrement apparaît alors complètement déformée.

Ces déformations sont d'autant plus gênantes que l'on tend de plus en plus vers une analyse automatique des courbes de variation de pression sanguine, de telles analyses prenant en compte non seulement la fréquence et l'amplitude des variations, mais aussi la dérivée de ces variations, grandeur qui est la plus perturbée par les phénomènes parasites de résonance.

Il peut encore survenir au contraire des phénomènes d'amortissement dus par exemple à la présence de sang ou de bulles d'air dans le cathéter ou du diamètre insuffisant de celui-ci. Ici encore, la courbe enregistrée est déformée, ce qui fausse les résultats de l'investigation, surtout si l'on prend en compte la dérivée de la courbe relevée.

Il est certain qu'un praticien exercé saura en général déceler à simple observation de la courbe enregistrée si elle est perturbée par une résonance ou un amortissement. Mais il ne pourra déceler le défaut qu'après une première mesure, c'est-à-dire une fois le cathéter engagé dans le vaisseau ou l'organe à explorer, et il faudra alors retirer le cathéter pour le changer ou le purger. Ceci oblige à une nouvelle introduction, toujours délicate, et qui prolonge d'autant la durée de l'intervention.

Il est donc très important pour le praticien de pouvoir tester l'ensemble du système de mesure, y compris le cathéter rempli de liquide, immédiatement avant de l'introduire dans le vaisseau du patient, c'est-à-dire dans la salle d'opération. Il faut pour un tel essai soumettre le système à des pressions variables, d'amplitude constante et à fréquences variant dans toute la plage des fréquences susceptibles d'être relevées chez le patient, et vérifier que dans cette plage le système ne présente ni résonance ni amortissement.

On connaît actuellement des appareils pour engendrer de telles pressions variables dans une capacité sur laquelle on pourra brancher le cathéter avant son introduction dans l'organisme du patient.

De tels générateurs sont normalement constitués par un simulateur électronique à fréquence variable, associé à un piston alternatif qui, en faisant varier le volume interne d'une capacité remplie d'un fluide, en fait varier la pression avec la même fréquence. Mais de tels appareils sont encombrants et complexes, et surtout se prêtent mal à une stérilisation complète et fréquente, et il n'est pas facile de les mettre à la disposition du praticien dans la salle d'opération même. Dans le document FR-A-1 556 606 on mentionne un générateur d'impulsions de pression pneumatiques périodiques dont la fréquence de pulsations est variable, comportant un conduit ayant un orifice d'entrée pour raccordement à une source à pression constante, un orifice de sortie où l'on recueille la pression variable et un orifice intermédiaire de fuite à débit contrôlé.

L'invention concerne en premier lieu un procédé de vérification d'un système de mesure de la pression et des variations de la pression intravasculaire d'un liquide organique dans un vaisseau, en vue de déterminer si le système présente des fréquences propres de résonance ou des phénomènes d'amortissement dans la plage de fréquences considérée, ce système comportant un cathéter et un appareil de mesure et d'enregistrement proprement dit, caractérisé par les étapes suivantes :

on raccorde le cathéter, rempli du liquide et destiné à être introduit dans le vaisseau, à un orifice de sortie d'un appareil générateur d'une pression fluide variable dont la fréquence des variations est elle-même variable, l'appareil générateur d'une pression fluide variable ayant été au préalable raccordé à la source à pression constante,

on lance en rotation l'appareil générateur de pression fluide variable,

on observe l'enregistrement des variations de pression aux fréquences croissantes, puis décroissantes au fur et à mesure de l'accélération, puis du ralentissement dudit appareil générateur de pression fluide variable, de façon à déceler les variations d'amplitude caractéristiques d'éventuels amortissements ou d'éventuelles résonances.

La présente invention permet aussi la réalisation d'un appareil simple et léger, aussi facilement stérilisable que les objets usuels utilisés en salle d'opération, et susceptible d'être ainsi constamment à portée de main du praticien pendant son intervention.

L'invention s'applique à un appareil générateur d'une pression fluide variable, à fréquence de variation elle-même variable, pour réaliser le procédé selon la revendication 1, comportant un conduit ayant un orifice d'entrée pour raccordement à la source à pression constante, un orifice de sortie où l'on recueille la pression variable, et un orifice intermédiaire de fuite à débit contrôlé, caractérisé en ce qu'il comporte en outre

une came rotative avec volant d'inertie, et des moyens de lancement en rotation ainsi que

des moyens mobiles de fermeture au moins partielle de l'orifice de fuite liés à la came rotative de façon à créer au moins un cycle ouverture-fermeture à chaque tour de la came.

Selon une forme particulière de réalisation de l'invention, les moyens mobiles de fermeture de l'orifice de fuite sont constitués par une aiguille montée élastiquement sur le socle de l'appareil, de manière à venir obturer l'orifice de fuite, l'aiguille prenant par ailleurs appui sur la came montée sur l'axe du volant de telle sorte que, selon les positions angulaires de la came au cours de sa rotation, l'aiguille soit plus ou moins repoussée et laisse ainsi l'orifice de fuite plus ou moins ouvert.

Selon une autre forme particulière de réalisation, la came est constituée par la partie périphérique du volant, le volant étant disposé de telle sorte que sa partie périphérique se déplace au voisinage immédiat de l'orifice de fuite de façon à modifier périodiquement le débit de fuite.

L'invention sera mieux comprise en se référant à des modes de réalisation particuliers, donnés à simple titre d'exemple et représentés par les dessins annexés.

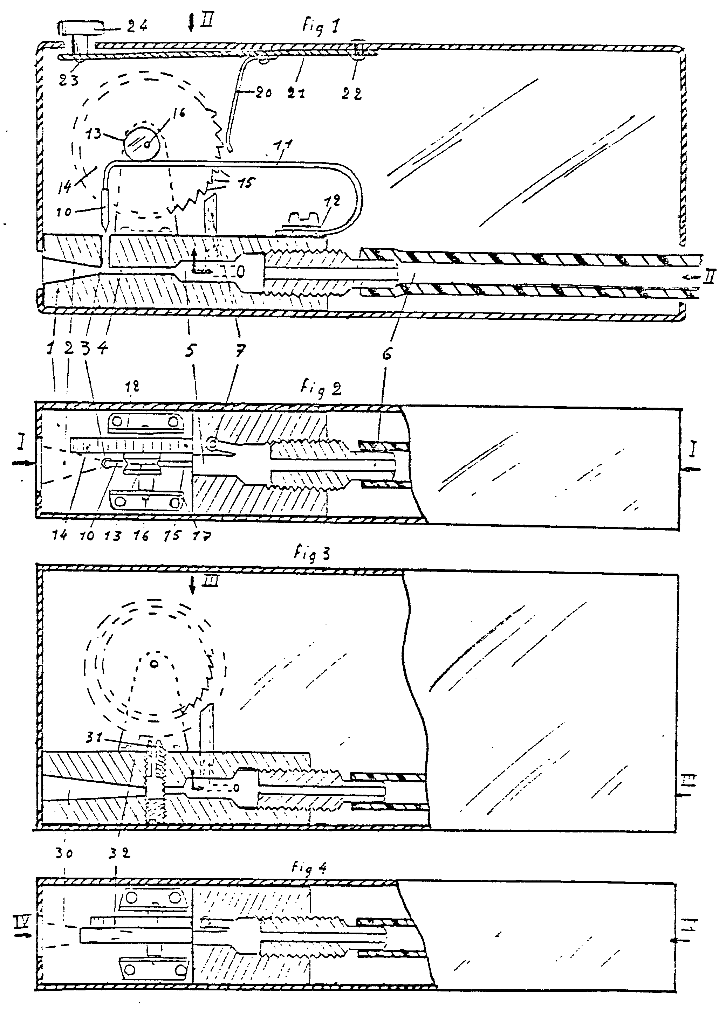

Les figures 1 et 2 de la planche unique concernent un appareil utilisant une aiguille de fermeture de l'orifice de fuite ;

la figure 1 est une vue en coupe de l'appareil suivant I-I.

la figure 2 est une vue de dessus, le boîtier étant supposé enlevé et l'appareil coupé partiellement suivant II-II.

Les figures 3 et 4 concernent un appareil utilisant le volant lui-même comme came d'obturation de l'orifice de fuite.

la figure 3 est une vue en coupe suivant IV-IV.

la figure 4 est une vue de dessus, le boîtier étant supposé enlevé et l'appareil coupé partiellement suivant III-III.

En se référant à l'ensemble des figures, on verra que l'appareil est constitué d'un bloc percé de différents orifices ou taraudages servant de support aux paliers du volant et monté dans un boîtier de prise en main de forme non spécifique dans le cas présent.

Le bloc 1 est percé d'un alésage conique 2 (servant à raccorder le cathéter) dont l'extrémité de petit diamètre communique d'une part avec l'ajutage à fuite contrôlée 3 et d'autre part, avec l'ajutage à fuite constante 4.

L'autre extrémité de l'ajutage à fuite constante 4 communique avec un conduit 5 raccordé à la source de fluide à pression constante 6. Ce même conduit 5 communique avec un ajutage 7 disposé de telle sorte qu'il dirige un jet de gaz sur les aubes de la turbine 14.

On comprend facilement que la pression dans l'alésage conique 2 (lorsqu'il est obturé par le cathéter dans lequel on désire générer une pression) dépend de la pression régnant dans le conduit 5 d'une part, et du rapport des résistances de fuite des ajutages 3 et 4 d'autre part. La résistance de fuite de l'ajutage 4 est constante tandis que celle de l'ajutage 3 varie entre l'infini (ajutage fermé hermétiquement) et la valeur déterminée par l'ajutage lui-même à pleine ouverture.

Les valeurs intermédiaires dépendent de l'obturation plus ou moins grande de la fuite 3.

Cette fuite 3 est obturée plus ou moins par une aiguille 10 de forme appropriée à la loi de variation de pression désirée solidaire de la tige élastique 11 fixée en 12 sur le socle. La tige élastique 11 est maintenue par son élasticité propre en contact léger avec la came 13 solidaire du volant 14 muni d'aubes 15 à sa périphérie, tournant fou dans l'axe 16 monté sur les supports 17 et 18. La rotation de la came 13 amènera la tige 11 à effectuer un mouvement de va-et-vient suivant une loi de variation dépendant de la forme propre de la came. Il sera facile en particulier d'obtenir un mouvement très sensiblement alternatif et sinusoïdal en utilisant une came constituée par un simple disque excentré.

En position de repos, le volant 14 est immobilisé par une lame 20 solidaire d'une seconde lame souple 21, fixée par une extrémité en 22 à l'intérieur du boîtier par exemple. L'autre extrémité 23 porte un bouton dépassant à l'extérieur du boîtier 24. On comprend qu'une pression sur le bouton 24 fera ployer la lame souple 21 et écartera la lame 20 du volant qui se trouvera libéré.

Si on applique simultanément une certaine pression fluide dans le conduit 5 par l'intermédiaire du tuyau 6 relié à la source de pression, l'ajutage 7 disposé tangentiellement au volant 14 agira pour le faire tourner par action sur les aubes 15. Le lancement en rotation du volant s'effectue donc tout simplement en le libérant par pression sur le bouton 24.

On pourra noter la grande simplicité de construction et d'utilisation de l'appareil ainsi réalisé, ce qui permet d'en faire un accessoire à la portée permanente du chirurgien en salle d'opération après stérilisation par les moyens usuels.

Lorsque le praticien s'apprête à effectuer une mesure de pression sanguine intravasculaire, l'appareil de mesure et d'enregistrement est raccordé à un cathéter rempli de liquide, comme du sérum physiologique. Pour vérifier l'ensemble du système de mesure, avant d'introduire le cathéter dans un vaisseau du patient, il suffit de raccorder l'extrémité du cathéter dans l'orifice 2 de l'appareil qui aura été, au préalable, raccordé par l'orifice 6 à une source de gaz comprimé à pression stable, air ou oxygène par exemple, usuellement disponible dans une salle d'opération.

La forme conique de l'orifice 2 permet le raccordement sans réglage préalable de cathéters de différents diamètres. On appuie ensuite sur le bouton 24. ce qui a pour effet de libérer le volant 14 qui sera alors entraîné en rotation par le jet de fluide provenant de l'ajutage 7. Le volant 14 solidaire de la came 13 entraînera le déplacement alternatif de l'aiguille 10. La variation périodique de la section de fuite de l'ajutage 3 entraîne une variation périodique de pression dans l'orifice 2. Le cathéter et l'appareil de mesure sont ainsi soumis à des pressions variables d'amplitude constante, dont la fréquence de variation croît lors du lancement du volant, puis décroît lorsque le volant est freiné par relâchement du bouton.

On couvrira une plage de fréquence plus large que celle pouvant être observée sur le patient, et si l'enregistrement d'essai obtenu avec l'appareil générateur de pression ne présente pas de perturbations dues à une résonance ou un amortissement, on pourra introduire le cathéter dans le vaisseau du patient sans risque de voir ensuite apparaître de telles perturbations.

L'appareil décrit aux figures 3 et 4 est une variante de réalisation qui s'utilise de la même façon que celui des figures 1 et 2. Mais, ici, le bloc support est percé de façon différente, de telle sorte que l'orifice conique de sortie 30 communique avec l'ajutage de fuite 31 qui débouche à proximité immédiate de la périphérie du volant 32. La périphérie lisse du volant 32 passe à proximité immédiate de l'orifice de fuite 31 ; ainsi, quand le volant tourne, sa périphérie s'écarte et se rapproche alternativement de l'orifice 31 en faisant varier périodiquement le débit de fuite, et en faisant varier avec la même fréquence la pression dans l'orifice 30 où sera raccordé le cathéter. Un volant circulaire excentré produira une variation de pression suivant une loi alternative sensiblement sinusoïdale mais on pourra naturellement imaginer d'autres formes de volant produisant des lois de variations différentes.

Bien entendu, l'invention n'est pas strictement limitée aux modes de réalisation qui ont été décrits à titre d'exemple, mais elle couvre également les réalisations qui n'en différeraient que par des détails, par des variantes d'exécution ou par l'utilisation de moyens équivalents. C'est ainsi que l'on pourrait utiliser tout autre système pour lancer le volant en rotation, par exemple un simple lancement à la main en agissant sur une partie faisant saillie, ou bien un lancement par tout système de ressort ou de moteur électrique ou autres.

L'invention peut s'appliquer dans les mêmes conditions à des vérifications sur tous systèmes de transport de fluides susceptibles de présenter des phénomènes de résonance ou d'amortissement. Bien entendu, les dimensions, les fréquences et les pressions engendrées seront adaptées au système.

**Revendications**

1. Procédé de vérification d'un système de mesure de la pression et des variations de la pression intravasculaire d'un liquide organique dans un vaisseau, en vue de déterminer si le système présente des fréquences propres de résonance ou des phénomènes d'amortissement dans la plage de fréquences considérée, ce système comportant un cathéter et un appareil de mesure et d'enregistrement proprement dit, procédé caractérisé par les étapes suivantes :

on raccorde le cathéter, rempli du liquide et destiné à être introduit dans le vaisseau, à un orifice de sortie d'un appareil générateur d'une pression fluide variable dont la fréquence des variations est elle-même variable, l'appareil générateur d'une pression fluide variable ayant été au préalable raccordé à une source à pression constante,

on lance en rotation l'appareil générateur de pression fluide variable,

on observe l'enregistrement des variations de pression aux fréquences croissantes, puis décroissantes au fur et à mesure de l'accélération, puis du ralentissement dudit appareil générateur de pression fluide variable, de façon à déceler les variations d'amplitude caractéristiques d'éventuels amortissements ou d'éventuelles résonances.

2. Appareil générateur d'une pression fluide variable, dont la fréquence de variation est elle-même variable, pour réaliser le procédé selon la revendication 1, comportant un conduit (5) ayant un orifice d'entrée (6) pour raccordement à la source à pression constante, un orifice de sortie (4) où l'on recueille la pression variable et un orifice intermédiaire de fuite à débit contrôlé (3), caractérisé en ce qu'il comporte en outre

une came rotative (13) avec volant d'inertie (14), et des moyens de lancement en rotation (24) ainsi que

des moyens mobiles (10-11) de fermeture au moins partielle de l'orifice de fuite (3) liés à la came rotative (13) de façon à créer au moins un cycle ouverture-fermeture à chaque tour de la came (13).

3. Appareil selon la revendication 2, caractérisé par le fait que les moyens mobiles de fermeture de l'orifice de fuite sont constitués par une aiguille (10) montée élastiquement sur le bloc support (1) de l'appareil de manière à venir obturer plus ou moins l'orifice de fuite (3), l'aiguille (10) prenant par ailleurs appui sur la came (13) montée sur l'axe (16) du volant (14) de telle sorte que, selon les positions angulaires de la came (13) au cours de la rotation, l'aiguille (10) soit plus au moins repoussée et laisse ainsi l'orifice de fuite (3) plus ou moins ouvert.

4. Appareil selon la revendication 2, caractérisé par le fait que la came (13) est constituée par la partie périphérique du volant (14), le volant (14) étant disposé de telle sorte que sa partie périphérique se déplace au voisinage immédiat de l'orifice de fuite (3) de façon à modifier périodiquement le débit de fuite.

**Claims**

1. Method of checking on a system for measuring the pressure and variations in the intravascular pressure of an organic fluid within a vessel, for the purpose of establishing whether the system incorporates natureal resonance frequencies or damping actions within the range of frequencies in question, this system comprising a catheter and a measuring and recording device as such, being a method characterised by the following stages :

the catheter filled with the fluid and intended to be inserted into the vessel is connected to an outlet opening of a device generating a variable fluid pressure whereof the very frequency of the variations is itself variable, the device generating a variable fluid pressure having previously been connected to a constant pressure source,

the device for generating a variable fluid pressure is placed in rotation,

the recording of the pressure variations at the frequencies increasing and then decreasing in step with the acceleration and then with the deceleration of the said device for generating a variable fluid pressure, is observed in a manner such as to detect the amplitude variations characteristic of possible damping actions or of possible resonance effects.

2. Device for generating a variable fluid pressure, whereof the very frequency of the variations is itself variable, for implementing the method according to claim 1, comprising a passage (5) having an inlet orifice (6) for connection to the constant pressure source, an outlet orifice (4) at which the variable pressure is picked up, and an intermediate leakage opening having a monitored rate of flow (3), characterised in that it also comprises

a rotary cam (13) having a flywheel (14) and means of rotational entrainment (21), as well as

movable means (10-11) for at least partial closure of the leakage opening (3) coupled to the rotary cam (13) in such manner as to engender at least one opening-closing cycle during each revolution of the cam (13).

3. Device according to claim 2, characterised by the fact that the movable means of closing the leakage opening comprise a needle (10) installed elastically on the supporting block (1) of the device in such manner as to shut off the leakage opening (3) to a greater or lesser extent, the needle (10) furthermore bearing on the cam (13) installed on the shaft (16) of the flywheel (14), in a manner such that as a function of the angular positions of the cam (13) in the course of rotation, the needle (10) is thrust back to a greater or lesser extent and thereby leaves the leakage opening (3) open to a greater or lesser extent.

4. Device according to claim 2, characterised by the fact that the cam (13) is formed by the peripheral section of the flywheel (14), the flywheel (14) being so arranged that its peripheral section is displaced in direct proximity to the leakage opening (3) in such manner that the leakage flow is modified periodically.

**Patentansprüche**

1. Verfahren zur Kontrolle eines Systems zur Messung des Drucks und der Änderungen des intravaskulären Drucks einer organischen Flüssigkeit in einem Gefäß zwecks Feststellung, ob das System Eigenresonanzfrequenzen oder Dämpfungserscheinungen im Bereich der betrachteten Frequenzen aufweist, welches System einen Katheter und eine eigentliche Meß- und Registriereinrichtung aufweist, gekennzeichnet durch die folgenden Schritte :

man verbindet den Katheter, der mit der Flüssigkeit gefüllt und zur Einführung in das Gefäß bestimmt ist, mit einer Ausgangsöffnung eines Apparats zur Erzeugung eines variablen Fluiddrucks, dessen Frequenz der Änderungen selbst variabel ist, welcher Apparat zur Erzeugung eines variablen Fluiddrucks vorab mit einer Konstantdruckquelle verbunden wurde,

man versetzt den Apparat zur Erzeugung eines variablen Fluiddrucks in Drehung,

man beobachtet die Aufzeichnung der Druckänderungen bei den wachsenden, dann sinkenden Frequenzen im Lauf der Beschleunigung, dann der Verzögerung des Apparats zur Erzeugung eines variablen Fluiddrucks derart, um die charakteristischen Amplitudenänderungen eventueller Dämpfungen oder eventueller Resonanzen nachzuweisen.

2. Vorrichtung zur Erzeugung eines variablen Fluiddrucks, dessen Änderungsfrequenz selbst variabel ist, zur Durchführung des Verfahrens nach Anspruch 1, mit einer Leitung (5) mit einer Eingangsöffnung (6) zum Anschluß an die Konstantdruckquelle, einer Ausgangsöffnung (4), wo man den variablen Druck erfaßt, und einer Leckzwischenöffnung (3) gesteuerten Durchsatzes, dadurch gekennzeichnet, daß sie außerdem eine drehbare Nockenscheibe (13) mit Trägheitsschwungmasse (14) und Mitteln (24) zum Versetzen in Drehung sowie bewegliche Mittel (10-11) zum wenigstens teilweisen Schließen der Lecköffnung (3) aufweist, die mit der drehbaren Nockenscheibe (13) derart verbunden sind, daß wenigstens ein Öffnungs-Schließungs-Zyklus bei jeder Umdrehung der Nockenscheibe (13) erzeugt wird.

3. Vorrichtung nach Anspruch 2, dadurch gekennzeichnet, daß die beweglichen Mittel zum Schließen der Lecköffnung durch eine Nadel (10) gebildet sind, die elastisch auf dem Trägerblock (1) der Vorrichtung so montiert ist, daß sie die Lecköffnung (3) mehr oder weniger absperrt, wobei die Nadel (10) im übrigen auf der Nockenscheibe (13), die auf der Achse (16) der Schwungmasse (14) montiert ist, derart abgestützt ist, daß die Nadel (10) je nach den Winkelstellungen der Nockenscheibe (13) im Lauf der Drehung mehr oder weniger zurückgestoßen wird und so die Lecköffnung (3) mehr oder weniger offen läßt.

4. Vorrichtung nach Anspruch 2, dadurch gekennzeichnet, daß die Nockenscheibe (13) durch den Umfangsteil der Schwungmasse (14) gebildet ist, wobei die Schwungmasse (14) so angeordnet ist, daß sich ihr Umfangsteil in unmittelbarer Nähe der Lecköffnung (3) derart vorbeibewegt, daß der Leckdurchsatz periodisch geändert wird.

0 049 723

Fig 1

Fig 2

Fig 3

Fig 4